**Europäisches Patentamt**

**European Patent Office**

**Office européen des brevets**

(11) Publication number: **0 078 267**
**B1**

## (12) EUROPEAN PATENT SPECIFICATION

(45) Date of publication of patent specification: **13.08.86**

(51) Int. Cl.⁴: **A 61 G 7/04**

(21) Application number: **82900109.8**

(22) Date of filing: **16.12.81**

(86) International application number:
**PCT/FI81/00092**

(87) International publication number:
**WO 82/01991 24.06.82 Gazette 82/16**

(54) **MATTRESS.**

(30) Priority: **16.12.80 FI 803924**

(43) Date of publication of application:
**11.05.83 Bulletin 83/19**

(45) Publication of the grant of the patent:
**13.08.86 Bulletin 86/33**

(84) Designated Contracting States:
**AT CH DE FR GB LI NL SE**

(56) References cited:
**FR-A-1 306 875**
**FR-A-1 464 593**
**US-A-1 097 894**
**US-A-3 670 345**

(73) Proprietor: **HYRY, Sirkka**
**Ahontie**
**SF-90310 Oulu 31 (FI)**

(72) Inventor: **HYRY, Sirkka**
**Ahontie**
**SF-90310 Oulu 31 (FI)**

(74) Representative: **Zipse + Habersack**
**Kemnatenstrasse 49**
**D-8000 München 19 (DE)**

Courier Press, Leamington Spa, England.

## Description

The present invention relates to a mattress for small children or incontinent persons having removably fitted in a region of the upper side thereof a changeable part having a moisture impermeable layer on the underside thereof.

Bed wetting is a serious problem, which in many cases leads to further complications such as cutaneous eruptions and other difficultly curable diseases, particularly as regards patients who are chronically ill and unable to move. For children and incontinent people it is customary to use moisture absorbing diapers, the purpose whereof is to absorb the urine. In this case, however, the skin of the patient is inconveniently fomented by the diaper. There has been an effort to reduce the fomenting effect of the diapers by employing so called dry diapers. The dry diaper consists of two separate layers, wherein the layer next to the skin is made of a highly liquid permeable material and the outer surface of a liquid absorbing material. Even with this construction, however, it has not been possible to eliminate the fomenting effect of the diaper completely. Moreover, the diaper is capable of absorbing only relatively small amounts of liquid.

In the prior art there are also known so-called baby mattresses, which are made of some suitable liquid absorbing material, for example acidic cellulose. The absorbing capacity of such mattresses is, however, very limited, and as can be understood from their title, they are meant mainly for the use of small babies.

FR—A—1 464 593 describes a mattress having an incision in its upper side allowing to insert a rectangular mattress element for reestablishing the planar surface. This mattress element comprises a moisture absorbing material having a moisture impermeable layer on the underside thereof and an upper permeable layer allowing the passage of the urine of a person lying on it. By employing such a mattress the child or incontinent person remains dry irrespective of urinating, if he is dressed in thin, permeable clothing, which is quickly dried up owing to the heat emanating from the skin. The mattress may also be kept clean only by changing the removable element. But especially with heavy persons there is the danger that the person, when moving presses the moisture back onto the surface of the mattress.

The purpose of the present invention is to realize such a mattress for small children, incontinent people etc., which eliminates the above mentioned drawbacks. In order to achieve this, the mattress of the present invention is characterized in that said mattress comprises a plurality of parallel grooves in said region arranged close to one another and said moisture impermeable layer of said changeable part is fitted into said grooves and that the grooves deepen at least part of the way towards a collecting groove which is formed in the mattress.

The mattress of the invention, provided with a plurality of parallel grooves for fitting the changeable part and connected with a collecting groove is particularly well suited for heavy patients. Thus, it can be ensured that the weight of the person does not press the moisture back onto the surface of the mattress, and unpleasant odours etc. can be avoided.

In the following the invention is described in detail with reference to the appended drawings.

Figure 1 is an axonometric illustration of a preferred embodiment of the invention;

Figure 2 shows the mattress of Figure 1 in lengthwise cross-section along the line B—B;

Figure 3 shows the mattress of Figure 1 in breadthwise cross-section along the line C—C.

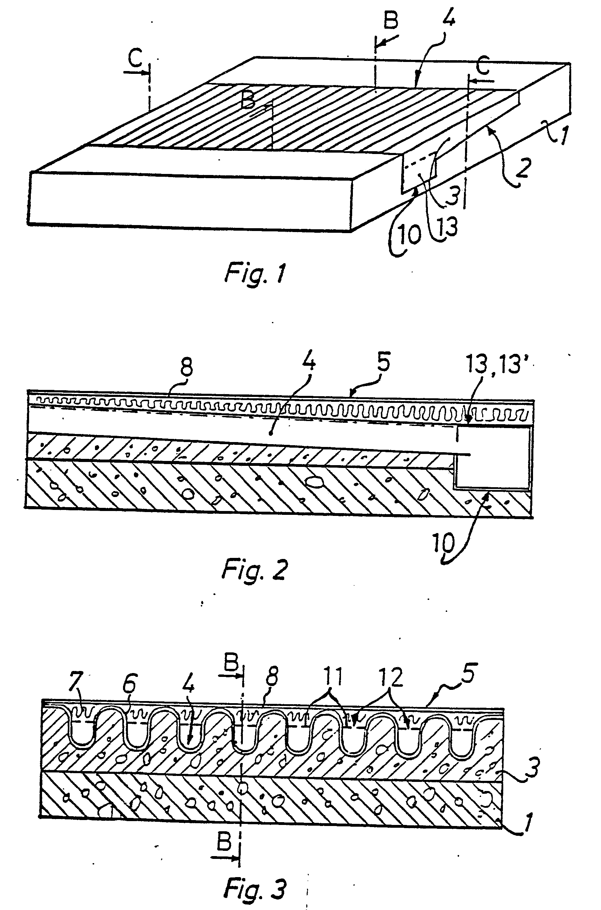

According to Figure 1, the mattress of the invention comprises a body 1 made for instance of foamed plastic. Therein is formed an incision 2. Into the incision 2 is fixedly fitted an element 3.

The detailed structure of the mattress is illustrated in Figure 3, which shows the mattress in breadthwise cross-section along the line C—C. The element 3 is provided with several parallel grooves 4. On top of the element 3 there is located a changeable part 5, so that it fits into the grooves 4. The changeable part 5 comprises the liquid impermeable layer 6, the form whereof matches the form of the grooves 4. At each groove 4 the part 5 is filled with liquid absorbing material 7 either entirely or according to Figure 3, only in the portion above tubular or hollow members with perforations 12 on their upper surfaces 11, which members can be matched into the grooves 4. The top layer 8 of the changeable part is in turn made of some highly liquid permeable material, such as fibrous cloth.

At one end of the incision 2 in the body 1, preferably at the foot of the bed, there is arranged a relatively wide collecting groove 10 in the crosswise direction. The parallel grooves 4 in element 3 become steadily deeper at least part of the way when moving from one end of the element 3 towards the crosswise collecting groove 10, as is clearly apparent from Figure 2.

The collecting groove 10 can be filled with a removable part 13 made of a moisture absorbing material, the part 13 being protected with a moisture impermeable layer at least on the bottom side. Another possibility is to fit into the collecting groove 10 a removable receptacle 13', which is at least partly closed. The grooves 4 are connected to the removable part 13 or to the receptacle 13'.

The above described mattress functions in the following manner. The moisture penetrates the changeable part 5 and flows through the perforations 12 into the tubular members located in the grooves 4. Along these tubular members the urine flows further into the removable receptacle or equivalent, situated in the crosswise collecting groove 10. When the receptacle becomes full, it is replaced by a new one. The top layer 8 of the changeable part is changed often, whereas the layer 6 can be changed after longer intervals.

In the above described embodiment the changeable part can be easily removed by gripping it at the edges and lifting it off the grooves of the mattress.

The most important thing is that the patient is securely not in contact with the moisture and that the patient's weight does not press the moisture back up through the changeable part. No such a danger exists with the mattress of Figures 1—3, which is suitably measured with respect to heavy patients in particular. The moisture impermeable layer 6 can be either a stationary member of the changeable part or removable therefrom.

The moisture permeable layer 8 is designed to form a substantially planar surface.

## Claims

1. A mattress for small children or incontinent persons having removably fitted in a region of the upper side thereof a changeable part (5) having a moisture impermeable layer (6) on the underside thereof, characterized in that said mattress comprises a plurality of parallel grooves (4) in said region arranged close to one another and said moisture impermeable layer of said changeable part is fitted into said grooves, and that the grooves (4) deepen at least part of the way towards a collecting groove (10) which is formed in the mattress.

2. The mattress of claim 1, characterized in that the changeable part (5) comprises a moisture absorbing part (7), which is located on the top of the moisture impermeable layer (6), at least at the points which are fitted into the grooves (4).

3. The mattress of claim 1 or 2, characterized in that the changeable part (5) is formed of tubular or hollow members with perforations (12) on their upper surfaces (11) at suitable intervals, and which members can be matched into the grooves (4).

4. The mattress of claim 1, 2 or 3, characterized in that within the collecting groove (10) there is a moisture absorbing changeable part (13) or a removable receptacle (13').

5. The mattress of any of the previous claims, characterized in that the changeable part (5) comprises a highly moisture permeable layer (8), which is arranged to be the uppermost layer, next to the patient's skin.

6. The mattress of claim 5, characterized in that the moisture permeable layer (8) forms a substantially planar surface.

7. The mattress of any of the previous claims, characterized in that the changeable part (5) forms part of an element (3) fitted into an incision (2) in the mattress body (1).

## Patentansprüche

1. Matratze für kleine Kinder oder Personen, die den Urin nicht halten können, die in einem Bereich der oberen Seite einen wechselbaren Teil (5) lösbar eingepaßt hat, der an der Unterseite eine feuchtigkeitsundurchlässige Schicht (6) hat, dadurch gekennzeichnet, daß die Matratze eine Vielzahl paralleler Nuten (4) in dem Bereich aufweist, die dicht beieinander angeordnet sind, und daß die feuchtigkeitsundurchlässige Schicht des wechselbaren Teils in die Nuten eingepaßt ist, und daß die Nuten (4) sich zumindest über einen Teil der Strecke zu einer sammelnden Nut (10) vertiefen, die in der Matratze ausgebildet ist.

2. Matratze nach Anspruch 1, dadurch gekennzeichnet, daß der wechselbare Teil (5) einen feuchtigkeitsabsorbierenden Teil (7) aufweist, der oben auf der feuchtigkeitsundurchlässigen Schicht (6) angeordnet ist, zumindest an den Punkten, die in die Nuten (4) eingepaßt sind.

3. Matratze nach Anspruch 1 oder 2, dadurch gekennzeichnet, daß der wechselbare Teil (5) aus rohrförmigen oder hohlen Gliedern mit Perforationen (12) an ihren oberen Oberflächen (11) in geeigneten Intervallen gebildet ist, wobei die Glieder in die Nuten (4) eingepaßt werden können.

4. Matratze nach Anspruch 1, 2 oder 3, dadurch gekennzeichnet, daß innerhalb der sammelnden Nut (10) ein feuchtigkeitsabsorbierender, wechselbarer Teil (13) oder ein entfernbares Gefäß (13') vorhanden ist.

5. Matratze nach einem der vorhergehenden Ansprüche, dadurch gekennzeichnet, daß der wechselbare Teil (5) eine hoch feuchtigkeitsdurchlässige Schicht (8) aufweist, die so angeordnet ist, daß sie die oberste Schicht, der Haut des Patienten benachbart ist.

6. Matratze nach Anspruch 5, dadurch gekennzeichnet, daß die feuchtigkeitsdurchlässige Schicht (8) eine im wesentlichen ebene Oberfläche bildet.

7. Matratze nach einem der vorhergehenden Ansprüche, dadurch gekennzeichnet, daß die wechselbare Teil (5) Teil eines Elements (3) bildet, welches in einen Einschnitt (2) im Matratzenkörper (1) eingepaßt ist.

## Revendications

1. Un matelas pour petits enfants ou personnes incontinentes présentant, montée de manière amovible dans une région de sa face supérieure, une partie pouvant être changée (5) présentant une couche (6) imperméable à l'humidité sur sa face inférieure caractérisé en ce que ledit matelas comprend un ensemble de gorges parallèles (4) dans ladite région disposées prés l'une de l'autre et en ce que ladite couche imperméable à l'humidité de ladite partie pouvant être changée est engagée dans lesdites gorges, et en ce que les gorges (4) deviennent en partie au moins plus profondes en direction d'une gorge collectrice (10) qui est formée dans le matelas.

2. Le matelas de la revendication 1, caractérisé en ce que la partie pouvant être changée (5) comprend une partie (7) absorbant l'humidité, qui est placée sur le dessus de la couche (6) imperméable à l'humidité, au moins au niveau des portions qui sont engagées dans les gorges (4).

3. Le matelas de la revendication 1 ou 2, caractérisé en ce que la partie (5) pouvant être changée est formée d'éléments tubulaires ou creux avec des perforations (12) sur leurs surfaces supérieures (11) à des intervalles appropriés, ces éléments pouvant être emboîtés dans les gorges (4).

4. Le matelas de la revendication 1, 2 ou 3, caractérisé en ce qu'à l'intérieur de la gorge collectrice (10) se trouve une partie pouvant être changée et absorbant l'humidité (13) ou un réceptacle amovible (13').

5. Le matelas de l'une quelconque des revendications précédentes, caractérisé en ce que la partie (5) pouvant être changée comprend une couche (8) fortement perméable à l'humidité et qui est prévue pour être la couche supérieure, près de la peau du patient.

6. Le matelas de la revendication 5, caractérisé en ce que la couche (8) perméable à l'humidité forme une surface sensiblement plane.

7. Le matelas de l'une quelconque des revendications précédentes, caractérisé en ce que la partie (5) pouvant être changée fait partie d'un élément (3) monté dans une incision (2) prévue dans le corps (1) du matelas.

Fig. 1

Fig. 2

Fig. 3